# EUROPÄISCHE PATENTSCHRIFT

(11) **EP 0 894 790 B1**
(45) Veröffentlichungstag und Bekanntmachung des Hinweises auf die Patenterteilung: **10.09.2003**
(21) Anmeldenummer: 98110854.1
(22) Anmeldetag: 13.06.1998
(51) Int. Cl.: C07D 211/70

(54) **Verfahren zur Herstellung von 1,2,3,6-Tetrahydro-2,2,6,6-tetramethylpyridin**
Process for the preparation of 1,2,3,6-tetrahydro-2,2,6,6-tetramethylpyridine
Procédé pour la préparation de 1,2,3,6-tétrahydro-2,2,6,6-tétraméthylpyridine

(30) Priorität: 29.07.1997 DE 19732589
(43) Veröffentlichungstag der Anmeldung: 03.02.1999
(73) Patentinhaber: Degussa AG, 40474 Düsseldorf (DE)
(72) Erfinder: Kaufhold, Manfred, Dr., 45770 Marl (DE); Jegelka, Udo, Dr., 45665 Recklinghausen (DE)

(56) Entgegenhaltungen:
- R. FRANKHAUSER; C.A. GROB; V. KRASNOBAJEW: "Synthese von 4-Chloropiperidinen" HELVETICA CHIMICA ACTA, Bd. 49, 1966, Seiten 690-695, XP002083680
- J. MARCH: "ADVANCED ORGANIC CHEMISTRY" 1985 , JOHN WILEY & SONS XP002083681 * Seite 901 - Seite 903 *
- D BORDEAUX ET AL: "Synthèse de radicaux libres nitroxydes dérivés du tétraméthyl-2,2,6,6 tétrahydropyridine-1,2,3,6;structure de l'epoxy-3,4 tétraméthyl-2,2,6,6 pipéridine-oxyle,C9H16NO2" ACTA CRYSTALLOGRAPHICA SECTION C. CRYSTAL STRUCTURE COMMUNICATIONS, Bd. 12, Nr. c39, 1983, Seiten 1656-1659, XP002074810
- HASSNER A ET AL: "A simple method of preparation of 7-alkyl-7 -azabicyclo[2.2.1]heptanes" TETRAHEDRON LETTERS, Bd. 36, Nr. 10, 6. März 1995, Seite 1709-1712 XP004028571

## Beschreibung

Die Erfindung betrifft ein Verfahren zur Herstellung von 1,2,3,6-Tetrahydro-2,2,6,6-tetramethylpyridin (im folgenden als "THTMP" bezeichnet) aus 4-Hydroxy-2,2,6,6-tetramethylpiperidin (im folgenden als "TAA-ol" bezeichnet).

THTMP ist von erheblichem Interesse, weil daraus das technisch wichtige 1,2,3,6-Tetrahydro-2,2,6,6-tetramethylpyridin-N-oxyl hergestellt werden kann. Ferner ist durch Hydrierung und anschließende Oxidation das 2,2,6,6-Tetramethylpiperidin-N-oxyl, als "TEMPO" bekannt, leicht zugänglich geworden. Beide N-Oxyle dienen als Polymerisationsinhibitoren für Monomere, wie Acrylsäure und deren Ester.

Synthesen von THTMP durch Dehydratisierung von TAA-ol sind aus der Literatur bekannt. E. Fischer gelang diese Reaktion in guter Ausbeute mit konzentrierter Schwefelsäure (Chem.Ber. 16 [1883]. 1604). Dabei wird jedoch ein großer Überschuß an Schwefelsäure benötigt, der nach der Reaktion neutralisiert werden muß. Mit konzentrierter Salzsaure entsteht THTMP aus TAA-ol bei höheren Temperaturen unter Druck neben der gewünschten Chlorverbindung, aus der durch Dehydrohalogenierung ebenfalls THTMP erhältlich ist (Fankhauser et al., Helv.Chim.Acta [49 [1966], 690).

Diese und weitere in der Literatur beschriebene Verfahren (Samtleben, Chem.Ber. 32 [1899], 644) haben einen hohen Verbrauch an Chemikalien, sind technisch aufwendig und mit Problemen der Abfallbeseitigung verbunden.

Aufgabe der Erfindung war es daher, ein Verfahren zu entwickeln, das ohne Verbrauch an Chemikalien auskommt und keine Probleme der Abfallbeseitigung bietet.

Diese Aufgabe wird durch ein Verfahren zur Herstellung von 1,2,3,6-Tetrahydro-2,2,6,6-tetramethylpyridin aus 4-Hydroxy-2,2,6,6-tetramethylpiperidin gelöst, bei dem man 4-Hydroxy-2,2,6,6-tetramethylpiperidin bei erhöhter Temperatur in der Gasphase an einem festen, schwach sauren Katalysator dehydratisiert.

Das Verfahren wird vorteilhaft kontinuierlich durchgeführt. Die Reaktion verläuft nach dem Formelschema

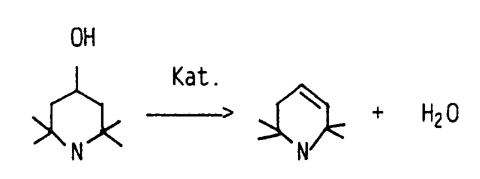

Es ist zwar bekannt, daß Alkohole in der Gasphase bei Temperaturen von 300 bis 400°C an festen Katalysatoren, wie Aluminiumoxid, Aluminiumphosphat, Thoriumoxid und Titandioxid, zu den entsprechenden Olefinen dehydratisiert werden können. Es ist weiter bekannt, daß man bei dieser Reaktion Isomerisierungen vermeiden kann, indem man ein Amin, z.B. Piperidin, zusetzt. Dessen Menge muß aber so gering wie möglich gehalten werden, weil es die sauren Zentren des Katalysators partiell vergiftet, wodurch dessen Aktivität und Laufzeit herabgesetzt werden (Organikum, Johann Ambrosius Barth-Verlag, 20. Auflage [1996], 262). Überraschenderweise gelingt die erfindungsgemäße Reaktion trotz der massiven Konzentration an NH-Gruppen im Ausgangsstoff TAA-ol.

Der Katalysator kann fest angeordnet, ein Fließ- oder Wirbelbettkatalysator sein oder gerührt werden. Geeignete Katalysatoren sind z.B. Kieselsäure (oder Siliciumdioxid), vorteilhaft zumindest teilweise in amorpher Form, und insbesondere saure Aluminiumoxide. Der saure Charakter sollte nicht zu stark ausgeprägt sein. Gegebenfalls kann man durch Zusatz von basischen Mitteln, z.B. Alkali- oder Erdalkalimetalloxiden oder -hydroxiden, oder von sauren Mitteln, wie Phosphorsäure, sauren Phosphaten oder Sulfaten, einen Katalysator optimieren. Durch orientierende Versuche kann man unschwer ermitteln, welche Mengen an basischen oder sauren Mitteln den Katalysator auf seine maximale Leistung bringen.

Bei Aluminiumoxiden kann man alternativ die Eignung als Katalysator in einfacher Weise feststellen, indem man eine bestimmte Menge, z.B. 10 g, in Wasser suspendiert und eine definierte Menge. z.B. 10 ml, 0,1 m Natronlauge zusetzt. Der pH steigt dann zunächst stark an, fällt aber im Verlauf von 0.6 bis 2 Stunden auf einen bestimmten, für das jeweilige Aluminiumoxid charakteristischen Endwert ab. Der pH-Abfall, d.h. die Differenz zwischen dem pH-Wert unmittelbar nach Zusatz der Natronlauge und dem Endwert, ist ein Maß für den sauren Charakter des Aluminiumoxids. Aluminiumoxide mit einem pH-Abfall um 0,5 bis 2,0 Einheiten, vorteilhaft um 0,6 bis 1,5 Einheiten sind gut als Katalysatoren geeignet. Aluminiumoxide mit geringerem pH-Abfall sind zu schwach sauer. Aluminiumoxide mit höherem pH-Abfall, wie 2,0 bis 3,5 Einheiten, sind stärker sauer, ergeben schlechtere Ausbeuten und werden daher nicht bevorzugt. Wie erwähnt, kann man zu schwach oder zu stark saure Katalysatoren durch Zusatz von sauren oder basischen Mitteln optimieren, also für ihren Einsatz in dem erfindungsgemäßen Verfahren verbessern oder überhaupt erst praktisch brauchbar machen. Dies gilt natürlich auch für Alumiumoxide.

Kieselsäure-Katalysatoren lassen sich, sofern sie nicht von Haus aus unmittelbar brauchbar sind, beispielsweise optimieren, indem man sie mit Phosphorsäure oder sauren Phosphaten dotiert.

Die Umsetzung wird in der Regel bei 200 bis 400°C, insbesondere bei 250 bis 350°C durchgeführt, je nach Katalysator und gewünschtem Durchsatz. Der Ausgangsstoff kann dem Katalysator in geschmolzener Form oder in Lösung zugeführt werden. Geeignete Lösungsmittel sind z.B. polare Lösungsmittel, wie N-Methylpyrrolidon. Auch Alkohole eignen sich als Lösungsmittel. Man erhält dann in einer Codehydratisierung neben dem gewünschten THTMP das dem Alkohol entsprechende Olefin. Weiterhin ist es möglich, den .Reaktionsaustrag als Lösemittel für den Ausgangsstoff zu verwenden und so in die Reaktion zurückzuführen, so daß kein reaktionsfremdes Lösungsmittel eingeführt werden muß. Die Schmelze oder Lösung des Ausgangsstoffes kann unmittelbar auf den Katalysator gegeben oder zuvor in einem üblichen Verdampfer verdampft und dem Katalysator dampfförmig zugeführt werden. Insbesondere bei einer Arbeitsweise ohne Lösungsmittel ist es empfehlenswert, zusammen mit dem Ausgangsstoff im Gleichstrom ein Inertgas, z.B. Stickstoff oder Argon, über den Katalysator zu leiten.

Das Verfahren kann z.B. durchgeführt werden, indem man den Ausgangsstoff geschmolzen (Fp. 130-132°C) oder gelöst auf den in einem senkrechten Rohrreaktor angeordneten erhitzten Katalysator gibt, wo er verdampft (Kp.₇₆₀ ca. 220°C). Alternativ leitet man den Ausgangsstoff oder dessen Lösung in einen Verdampfer und die Dämpfe auf den erhitzten Katalysator. Gleichzeitig wird ein Inertgasstrom von oben über den Katalysator geleitet, der Ausgangsstoff und Produkt zum unteren Ende der Reaktionszone fördert. Man überwacht den Umsatz, indem man im Reaktionsaustrag gaschromatographisch TAA-ol bestimmt, und regelt den Durchsatz entsprechend. Ein Durchsatz der 0.1- bis 1-fachen, vorteilhaft der 0,2 bis 0.8-fachen Gewichtsmenge Ausgangsstoff, bezogen auf den Katalysator, hat sich bewährt. Das Reaktionsgemisch wird nach dem Austritt aus dem Reaktor durch Kühlen verflüssigt. Aus dem Gasstrom können mitgeführte kondensierbare Anteile in einer Kühlfalle abgeschieden werden.

Die folgenden Beispiele sollen die Erfindung weiter erläutern, nicht jedoch ihren Anwendungsbereich begrenzen, wie er in den Patentansprüchen dargelegt ist.

### Beispiel 1

Man benutzt eine Glasapparatur, die aus einem senkrechten, elektrisch beheizbaren Rohr von 50 cm Länge und 3 cm Durchmesser, einem beheizbaren Tropftrichter und eine Vorlage mit Eisbad besteht. Das Rohr wird mit 150 g Aluminiumoxid (SPHERALITE^{(R)}SP 508 F der Fa. PROCATA-LYSE. Falinders, Frankreich; extrudierte Stränge von 1.6 mm Durchmesser) gefüllt. Der Katalysator zeigt in dem zuvor beschriebenen Test einen pH-Abfall von 0.9 Einheiten. Der Tropftrichter wird mit TAA-ol rein (99,5%) gefüllt und auf 160°C erwärmt. Nachdem das TAA-ol geschmolzen ist, wird es in einer Menge von 75 ml/h auf den auf 280°C erhitzten Katalysator getropft. Gleichzeitig wird ein Stickstoffstrom von 20 l/h von oben über den Katalysator geleitet. Nach 24 Stunden hat der Katalysator fast seine volle Aktivität erreicht. Danach werden bei einer Reaktionstemperatur zwischen 300 und 315°C 1.332 g TAA-ol mit konstanter Geschwindigkeit von 75 ml/h über den Katalysator gefahren und 1.221 g Reaktionsaustrag erhalten. Zur Aufarbeitung wird zunächst mit Cyclohexan als Schleppmittel durch azeotrope Destillation Wasser ausgekreist. Die fraktionierte Destillation ergibt THTMP in einer Ausbeute von ca. 70 % d.Th..

### Beispiel 2

Man arbeitet wie im Beispiel 1, verwendet jedoch ein vergleichsweise stärker saures Aluminiumoxid (SPHERALITE^{(R)}SP 521 E; pH-Abfall 2,9). Die Ausbeute an THTMP beträgt nur 15% d.Th.. Es entstehen hauptsächlich leicht siedende Spaltprodukte.

### Beispiele 3 bis 5

Man arbeitet wie im Beispiel 1 und verwendet das vergleichsweise stärker saure Aluminiumoxid SPHERALITE^{(R)}SP 521 E des Beispiels 2, das jedoch zuvor mit Natriumhydroxid-, Strontiumhydroxid- bzw. Bariumhydroxidlösung getränkt und getrocknet worden war, so daß der Gehalt an dem jeweiligen Hydroxid, gerechnet als Oxid, 1 Gew.-% betrug. Diese drei Katalysatoren ergaben die folgenden Ausbeuten:

| | | |
|---|---|---|
| Beispiel 3 | SP 521 E mit 1% Na₂O | ca. 25% d.Th. |
| Beispiel 4 | SP 521 E mit 1% SrO | ca. 60% d.Th. |
| Beispiel 5 | SP 521 E mit 1% BaO | ca. 58% d.Th. |

### Beispiel 6

Man benutzt als Reaktor ein Quarzrohr mit einem Durchmesser von 35 mm und einer Länge von 80 cm, das mit einem elektrischen Ofen beheizt wird. Der Reaktor wird mit 202 g des in Beispiel 1 verwendeten sauren Aluminiumoxids SPHERALITE^{(R)}508 F, das jedoch, wie in den Beispielen 3 bis 5 beschrieben, mit 0.5 Gew.-% BaO dotiert wurde, mittig gefüllt. Unterhalb und oberhalb des Katalysators befinden sich Quarzsplitter.

Im Reaktor wird eine Temperatur von 330°C eingestellt. TAA-ol roh (95 %) wird über einen bei 260°C betriebenen Verdampfer gasförmig oben in den Reaktor eingeleitet. Der Durchsatz beträgt 180 ml TAA-ol roh pro Stunde, gleichzeitig werden 1,5 l/h Stickstoff durch den Verdampfer und den Reaktor geleitet. Die Ausbeute an THTMP steigt in den ersten Stunden stark an und pendelt sich nach etwa 100 Stunden auf ca 82% d.Th. ein. Auch nach einer Woche besitzt der Katalysator noch die gleiche Aktivität.

### Vergleichsbeispiel 1

Man arbeitet wie im Beispiel 1, verwendet jedoch ein neutrales Aluminiumoxid (SPHERALITE^{(R)}SP 512; pH-Abfall <0,1). Bei der Reaktionstemperatur von 300 bis 315°C entsteht kein THTMP. Wenn man die Temperatur um 50°C steigert, erhält man bei einem TAA-ol-Umsatz von 33% eine Ausbeute an THTMP von <1% d.Th..

### Beispiel 7

Man arbeitet wie im Beispiel 1, verwendet jedoch einen Katalysator. der aus teils amorpher und teils kristalliner Kieselsäure besteht, die mit Phosphorsäure getränkt ist. Der Gehalt an Phosphorsäure beträgt 35 Gew.-%. Die mit diesem Katalysator erzielte Ausbeute an THTMP beträgt 45% d.Th..

### Vergleichsbeispiel 2

Man arbeitet wie im Beispiel 7, verwendet jedoch als Katalysator neutrale, amorphe Kieselsäure, nämlich das Kieselgel KC Siliperl^{(R)}AF 125 der Fa. Engelhard Industries. Die Ausbeute an THTMP beträgt <2% d.Th..

## Patentansprüche

1. Verfahren zur Herstellung von 1,2,3,6-Tetrahydro-2,2,6,6-tetramethylpyridin aus 4-Hydroxy-2,2,6,6-tetramethylpiperidin,
**dadurch gekennzeichnet,**
**dass** man 4-Hydroxy-2,2,6,6-tetramethylpiperidin bei 200 bis 400 °C in der Gasphase an einem festen sauren Katalysator dehydratisiert.

2. Verfahren nach Anspruch 1,
**dadurch gekennzeichnet,**
**dass** die Umsetzung kontinuierlich durchgeführt wird.

3. Verfahren nach Anspruch 1 oder 2,
**dadurch gekennzeichnet,**
**dass** als saurer fester Katalysator ein Aluminiumoxid eingesetzt wird, welches bei einem Test unter Einsatz einer bestimmten Aluminiumoxidmenge in Gramm und der zahlenmäßig gleichen Menge an 0,1 molarer Natronlauge in Milliliter innerhalb von 0,6 bis 2 Stunden einen pH-Abfall von 0.5 bis 2.0 Einheiten zeigt.

4. Verfahren nach Anspruch 1 oder 2,
**dadurch gekennzeichnet,**
**dass** als saurer fester Katalysator Kieselsäure verwendet wird, die mit Phosphorsäure oder sauren Phosphaten dotiert wurde.

5. Verfahren nach Anspruch 1 oder 2,
**dadurch gekennzeichnet,**
**dass** als saure feste Katalysatoren Kieselsäure oder Aluminiumoxid verwendet werden, die mit Alkali- oder Erdalkalimetalloxid oder -hydroxid dotiert wurden.

6. Verfahren nach Anspruch 1 oder 2,
**dadurch gekennzeichnet,**
**dass** als saure feste Katalysatoren Kieselsäure oder Aluminiumoxid verwendet werden, die mit Strontium- oder Bariumoxid oder -hydroxid dotiert wurden.

7. Verfahren nach Anspruch 1,
**dadurch gekennzeichnet,**
**dass** die Temperatur 250 bis 350 °C beträgt.

8. Verfahren nach einem der Ansprüche 1 bis 7,
**dadurch gekennzeichnet,**
**dass** man 4-Hydroxy-2,2,6,6-tetramethylpiperidin in einem polaren Lösungsmittel gelöst einsetzt.

9. Verfahren nach einem der Ansprüche 1 bis 7,
**dadurch gekennzeichnet,**
**dass** man 4-Hydroxy-2,2,6,6-tetramethylpiperidin in Reaktionsaustrag gelöst einsetzt.

10. Verfahren nach einem der Ansprüche 1 bis 9,
**dadurch gekennzeichnet,**
**dass** man die Umsetzung in Gegenwart eines Inertgases durchführt.

## Claims

1. A process for preparing 1,2,3,6-tetrahydro-2,2,6,6-tetramethylpyridine from 4-hydroxy-2,2,6,6-tetramethylpiperidine, **characterized in that** 4-hydroxy-2,2,6,6-tetramethylpiperidine is dehydrated at from 200 to 400°C in the gas phase over a solid acid catalyst.

2. A process according to claim 1, **characterized in that** the reaction is carried out continuously.

3. A process according to claim 1 or 2, **characterized in that** the solid acid catalyst used is an aluminium oxide which displays a pH drop of from 0.5 to 2.0 over a period of from 0.6 to 2 hours in a test using a particular amount of aluminium oxide in grams and the numerically equal amount of 0.1 molar aqueous sodium hydroxide solution in millilitres.

4. A process according to claim 1 or 2, **characterized in that** the solid acid catalyst used is silica which has been doped with phosphoric acid or acid phosphates.

5. A process according to claim 1 or 2, **characterized in that** the solid acid catalysts used are silica or aluminium oxide which have been doped with alkali metal or alkaline earth metal oxide or hydroxide.

6. A process according to claim 1 or 2, **characterized in that** the solid acid catalysts used are silica or aluminium oxide which have been doped with strontium or barium oxide or hydroxide.

7. A process according to claim 1, **characterized in that** the temperature is from 250 to 350°C.

8. A process according to any one of claims 1 to 7, **characterized in that** 4-hydroxy-2,2,6,6-tetramethylpiperidine is used in solution in a polar solvent.

9. A process according to any one of claims 1 to 7, **characterized in that** 4-hydroxy-2,2,6,6-tetramethylpiperidine is used in solution in the reaction product.

10. A process according to any one of claims 1 to 9, **characterized in that** the reaction is carried out in the presence of an inert gas.

## Revendications

1. Procédé de préparation de 1,2,3,6-tétrahydro-2,2,6,6-tétraméthylpyridine à partir de 4-hydroxy-2,2,6,6-tétraméthylpipéridine,
**caractérisé en ce qu'**
on déshydrate la 4-hydroxy-2,2,6,6-tétraméthylpipéridine entre 200 et 400°C en phase gazeuse sur un catalyseur acide solide.

2. Procédé selon la revendication 1,
**caractérisé en ce qu'**
on effectue la réaction de façon continue.

3. Procédé selon la revendication 1 ou 2,
**caractérisé en ce qu'**
on utilise comme catalyseur acide solide un oxyde d'aluminium qui présente dans une épreuve utilisant une quantité déterminée d'oxyde d'aluminium en grammes et une quantité numériquement identique de lessive de soude 0,1 M en ml, en 0,6 à 2 heures, une baisse de pH de 0,5 à 2,0 unités.

4. Procédé selon la revendication 1 ou 2,
**caractérisé en ce qu'**
on utilise comme catalyseur acide l'acide silicique additionné d'acide phosphorique ou de phosphates acides.

5. Procédé selon la revendication 1 ou 2,
**caractérisés en ce qu'**
on utilise comme catalyseurs acides l'acide silicique ou l'oxyde d'aluminium qui sont additionnés d'oxydes ou d'hydroxydes de métaux alcalins ou alcalino-terreux.

6. Procédé selon la revendication 1 ou 2,
**caractérisé en ce qu'**
on utilise comme catalyseurs solides acides l'acide silicique ou l'oxyde d'aluminium qui a été additionné d'oxyde ou d'hydroxyde de strontium ou de baryum.

7. Procédé selon la revendication 1,
**caractérisé en ce que**
la température s'élève à 250 à 350°C.

8. Procédé selon l'une des revendications 1 à 7,
**caractérisé en ce qu'**
on utilise la 4-hyroxy-2,2,6,6-tétraméthylpipéridine dissoute dans un solvant polaire.

9. Procédé selon l'une des revendications 1 à 7,
**caractérisé en ce qu'**
on utilise la 4-hydroxy-2,2,6,6-tétraméthylpipéridlrie sous forme dissoute dans le produit de la réaction.

10. Procédé selon l'une des revendications 1 à 9,
**caractérisé en ce qu'**
on conduit la réaction en présence d'un gaz inerte.
